# EUROPEAN PATENT APPLICATION

(11) **EP 2 311 493 A1**
(43) Date of publication of application: **20.04.2011**
(21) Application number: 09750548.1
(22) Date of filing: 19.05.2009
(51) Int. Cl.: A61K 39/395, A01K 67/027, A61P 35/00, A61P 37/06, C07K 16/02, C12N 15/09

(54) **CYTOTOXIC COMPOSITION**

(30) Priority: 20.05.2008 JP 2008131596
(71) Applicant: Kaneka Corporation, Osaka-shi, Osaka 530-8288 (JP)
(72) Inventor: YAMASHITA, Takashi, Takasago-shi Hyogo 676-8688 (JP)
(74) Representative: Vossius & Partner
(86) International application number: PCT/JP2009/059161
(87) International publication number: WO 2009/142186

(57) **Abstract**

The present invention relates to a cytotoxic composition that contains, as an active ingredient, an IgG antibody produced in eggs of a transgenic avian with a gene encoding the IgG antibody. The IgG antibody is expected to produce high anti-tumor effects with less side-effects because this antibody has higher CDC activity than that of the corresponding CHO cell-derived IgG antibody and its primary protein structure is not altered. The present invention also provides an agent for ameliorating or treating a cancer and an agent for treating an autoimmune disease, which contain the cytotoxic composition.

## Description

### TECHNICAL FIELD

The present invention relates to a cytotoxic composition that contains, as an active ingredient, a recombinant IgG antibody with higher complement-dependent cytotoxicity (CDC) activity than that of the corresponding wild-type antibody and antibody produced by CHO cell culture.

### BACKGROUND ART

Antibodies are glycoproteins referred to as immunoglobulins and produced by a type of lymphocytes known as B cells. Antibodies inherently have a high ability to recognize specific molecular structures (antigens) and bind to them, and work to eliminate bacteria, viruses, and other non-self proteins invading living tissues, thus playing an important role against infection (Non-patent Document 1).

Techniques have been developed to prepare antibodies that are homogeneous in nature (monoclonal antibodies) from clones of single antibody-producing cells. The development of these techniques has enabled the practical use of antibody drugs targeting cancerous cells or specific antigens that may cause immune diseases. Typically, monoclonal antibodies are prepared by methods for preparing an antibody to an antigen of interest using mouse cells. In the case that monoclonal antibodies prepared by these methods are intended to be administered to humans, parts of the monoclonal antibodies, for example, constant regions thereof should be replaced with amino acid sequences of human antibodies in order to avoid rejection.

Antibody drugs can be effective drugs for cancers and autoimmune diseases because of their high specificity (Non-patent Document 2). However, the disadvantage thereof is high production costs, which leads to high medical costs. To overcome this disadvantage, improvement of the production methods has been desired. Animal factories for transgenic (recombinant) chickens and the like are one attempt to achieve production of low-cost antibodies (Patent Document 1).

As clinical data and knowledge of antibody drugs in practical use accumulated, it was found that these antibody drugs do not always provide satisfactory effects. Accordingly, there is a need for finding more effective antibodies. To increase the activity levels of antibodies for the purpose of increasing the efficacy thereof leads to the reduction of administration dose, resulting in lower costs, in addition to the advantage of higher efficacy.

In order to produce antibodies with higher activity, techniques are under investigation which increase antibody-dependent cell mediated cytotoxicity (ADCC) activity or complement-dependent cytotoxicity (CDC) activity by conversion of sugar chains or amino acids of antibodies by gene-recombination technology (Patent Document 2). As is known in the art, ADCC activity can be increased by removing fucose from the complex N-glycoside-linked sugar chain bound to the Fc region of antibodies. Non-Patent Document 3 teaches that an antibody produced in egg white of transgenic chickens by the expression of an introduced gene has complex N-glycoside-linked sugar chains with a low fucose content and hence high ADCC activity.

Although ADCC activity can be increased by such artificial conversion of sugar chains, there have been no established practical techniques to increase CDC activity. As described above, antibodies having no fucose in the complex N-glycoside-linked sugar chains bound to the Fc region are expected to have high ADCC activity. These antibodies, however, may not have high CDC activity. There are some subclasses of IgG antibodies. For antibody drugs, IgG1 is commonly used because IgG1 has high efficacy, and selectively binds to protein A and therefore is easily purified. However, it is not IgGl but IgG3 that has higher CDC activity. In fact, there are known techniques to artificially prepare an antibody drug with enhanced CDC activity by substituting part of the Fc region of an IgG1 antibody with amino acids of an IgG3 antibody. Such chimeric antibodies obtained by partial structure conversion between IgG subclasses are not natural substances and may provide unpredictable side-effects (antigenicity). Accordingly, there is a demand for techniques to enhance CDC activity without altering the primary protein structure of the Fc region of antibodies.

CDC activity plays an important role in efficiently eliminating target cells. Application of IgG antibodies with high CDC activity to anti-cancer antibody drugs or antibody drugs for ameliorating autoimmune diseases such as allergies and atopy is expected to provide remarkably high effects. CDC activity is deeply involved in the anticancer mechanism of rituximab, which is an antibody for treating non-Hodgkin's lymphomas (Non-Patent Document 4). Development of techniques to prepare IgG antibodies with high CDC activity at low cost without altering the amino acid structure of the corresponding wild-type antibodies will be good news for patients with cancers and autoimmune diseases, the prevalence of which is high today.

Patent Document 1: W02004/016081
Patent Document 2: W02007/011041

Non-Patent Document 1: Monoclonal Antibodies: Principles and Applications, Wiley-Liss, Inc. (1995)
Non-Patent Document 2: Nature Reviews Cancer, 1, 119 (2001)
   Non-Patent Document 3: Nature Biotechnology, 28, 1038 (2005)
      Non-Patent Document 4: Biochemi. Soc. Trans, 25: 705 (1997)

### SUMMARY OF THE INVENTION

There is a demand for an antibody composition having improved functions that result in therapeutic effects, such as high CDC activity and ADCC activity, without altering the primary protein structure of the corresponding wild-type antibody, and a method for producing the composition at low cost.

The present inventor found that an IgG antibody accumulated in an egg of a transgenic avian with a gene encoding the IgG antibody has higher CDC activity than that of the corresponding CHO cell-derived IgG antibody, and thus completed the present invention.

The present invention was completed based on the above knowledge and includes the following aspects:

(1) a cytotoxic composition containing an IgG antibody as an active ingredient, the IgG antibody being obtainable from an egg of a transgenic avian that contains a gene encoding the IgG antibody;
(2) the cytotoxic composition described in (1), wherein the active ingredient is an antibody obtainable from egg white;
(3) the cytotoxic composition described in (1) or (2), wherein the IgG antibody has a constant region of human IgG class;
(4) the cytotoxic composition described in any one of (1) to (3), wherein the transgenic avian is a transgenic poultry;
(5) the cytotoxic composition described in any one of (1) to (4), wherein the fucose content of complex N-glycoside-linked sugar chains of the antibody is not more than one fifth of that of the corresponding CHO cell-derived antibody;
(6) the cytotoxic composition described in any one of (1) to (5), wherein CDC activity of the antibody is five or more times higher than that of the corresponding CHO cell-derived antibody;
(7) the cytotoxic composition described in any one of (1) to (6), wherein the IgG antibody recognizes an antigen specifically expressed in a cancer cell;
(8) an agent for ameliorating or treating a cancer, containing the cytotoxic composition described in any one of (1) to (7);
(9) the cytotoxic composition described in any one of (1) to (6), wherein the IgG antibody neutralizes and inhibits an effector cell that reacts with an autoantigen; and
(10) an agent for ameliorating or treating an autoimmune disease, containing the cytotoxic composition described in any one of (1) to (6) and (9).

The cytotoxic composition of the present invention contains, as an active ingredient, an IgG antibody obtainable from an egg of a transgenic avian that contains a gene encoding the IgG antibody. The IgG antibody has higher CDC activity than that of the corresponding CHO cell-derived IgG antibody, and its primary protein structure is not altered from that of the corresponding wild-type antibody. Therefore, use of the IgG antibody in antibody drugs will provide higher effects with less side-effects. Also, the antibody is expected to have high ADCC activity based on the previous knowledge that antibodies produced in egg white of transgenic chickens have complex N-glycoside-linked sugar chains with a low fucose content. Since both of CDC activity and ADCC activity are high, the antibody can effectively destroy, kill or inactivate target cells.

Since the IgG antibody can be produced in an egg of a transgenic avian, the production costs will be low.

### BRIEF DESCRIPTION OF THE DRAWINGS

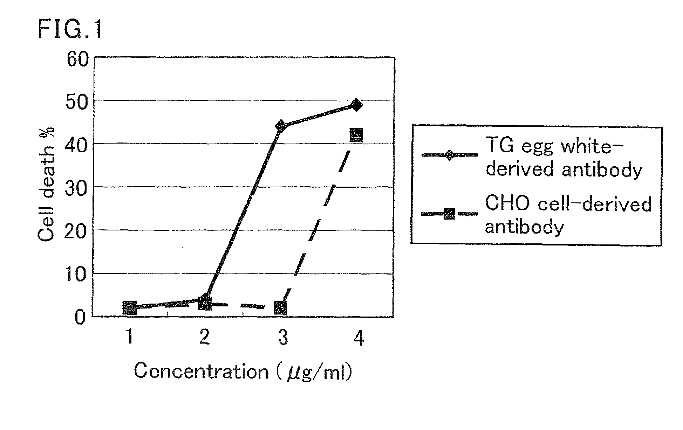

Fig. 1 is a graph of the results of Example 9 of the present invention, which illustrates the killing activity levels (CDC activity levels) of a transgenic chicken-derived fully human anti-TNF antibody and a CHO cell-derived fully human anti-TNF antibody, to TNF antigen-expressing T cells. The "TG egg white-derived antibody" in the graph corresponds to the fully human anti-TNF antibody purified from egg white of a transgenic chicken. The "CHO cell-derived antibody" in the graph corresponds to the fully human anti-TNF antibody produced in CHO cells. The vertical axis represents the mortality of target cells determined by FACS.

### BEST MODE FOR CARRYING OUT THE INVENTION

The cytotoxic composition of the present invention contains, as an active ingredient, an IgG antibody obtainable from an egg of a transgenic avian that contains a gene encoding the IgG antibody. The antibody has high CDC activity or has both high CDC activity and high ADCC activity, and therefore the composition can destroy, kill or inactivate cells involved in diseases and disorders of certain types.

The transgenic avian may be produced by any gene transfer technique. Examples thereof include virus-mediated gene transfer techniques, sperm-mediated gene transfer techniques, and techniques with use of PGC (primordial germ cell).

For safety reasons, a replication defective retroviral vector is preferably used in the present invention. Preferably, the retroviral vector is rendered replication defective by deleting at least a part or the whole of each coding sequence or each sequence necessary for the expression thereof so that one or a combination of the protein contained in the internal core (group specific antigen, gag), reverse transcriptase (polymerase, pol) and envelope glycoprotein (envelope, env), which are necessary for virus particle replication, may not be expressed, or by causing a mutation or mutations by substitution and/or insertion so that these proteins may not be expressed.

Since the length of a gene that is successfully inserted into the retroviral vector is limited depending on the viral species, deletion mutations are preferable. In order to ensure safety and increase the length of an insert fragment, it is preferable to delete two or more of the gag, pol and env regions. Preferably, the retroviral vector contains a viral packaging signal (phi), which functions as a signal for packaging of the vector in a virus particle. Since part of the gag region may function as a viral packaging signal, the viral vector preferably contains at least part of the gag region that is rendered incapable of being expressed, in order to increase the virus titer (J. Virol., 1987, 61(5), 1639-46).

The retrovirus is not particularly limited, and examples thereof include viruses derived from Moloney murine leukemia virus, Moloney murine sarcoma virus, avian leukemia virus (ALV), and human immunodeficiency virus (HIV). Viruses highly capable of infecting germ cells and stem cells, such as murine stem cell virus (MSCV) and murine embryonic stem cell virus (MESV), are preferably used for the infection of avian embryos. For effective infection of avian cells with such a viral vector, it is preferable to artificially replace the coat protein with the bovine vesicular stomatitis virus-derived VSV-G envelope protein. It should be noted that the envelope protein is not limited to those of viruses of this type

Marker genes are genes each encoding a protein serving as a marker for identification and isolation of a cell into which the desired gene is properly transferred. The retroviral vector may contain a marker gene. The marker gene is not particularly limited, and examples thereof include genes for fluorescence proteins such as green fluorescent protein (GFP), cyan fluorescent protein (GFP), and luciferase; drug resistance genes such as neomycin resistance gene (Neo^{r}), hygromycin resistance gene (Hyg^{r}), and puromycin resistance gene (Puro^{r}); and genes for thymidine kinase, dihydrophorate reductase, aminoglycoside phosphotransferase, chloramphenicol acetyltransferase, β-lactamase, and β**-**galactosidase. The marker gene is preferably accompanied by a promoter sequence and other elements necessary for the expression thereof.

A foreign gene containing the coding sequence of the antibody protein is used, and the limits of the coding sequence are defined by the initiation codon at the 5' terminal and the corresponding termination codon at the 3' terminal. The foreign gene preferably contains a Kozak consensus sequence near the initiation codon at the 5' terminal. The foreign gene preferably contains a ribosome binding site upstream of the coding sequence. Further, the foreign gene preferably contains at least part of or the whole of a suitable promoter sequence necessary for the expression thereof in avian cells. The term "promoter sequence" means a region on DNA or RNA which determines a transcription initiation site of a gene and directly regulates the frequency of transcription.

Antibody molecules have binding regions that are variable according to the structure of an antigen, and constant regions that are comparatively less variable. Antibody molecules are classified into several types (classes) based on the structures of their constant regions. Five different antibody classes (IgG, IgE, IgA, IgM, and IgD) each having a different constant region are known in mammals, and they differ in bioactivity. IgA and IgG are further classified into subclasses.

For antibody drugs, IgG1 subclass antibodies are used because of their long lifetime in blood and high efficacy. The antibody gene transferred into a transgenic avian in the present invention is preferably a structural gene of an IgG1 subclass antibody. IgG1 subclass antibodies are excellent in the accumulation efficiency in eggs of transgenic avians. Alternatively, structural genes of foreign antibodies such as genes of antibodies having a constant region of human IgG class, and genes of antibodies having a constant region of IgG subclass (e.g. human IgG1, quail IgG2, chicken IgG2, murine IgG2) are preferably used as the antibody gene since these antibodies show high accumulation in eggs.

Other preferable examples of the antibody structural gene include structural genes of interspecific chimeric antibodies. The term "interspecific chimeric antibodies" used herein means hybrid antibodies of the same IgG subclasses, produced based on gene sequences of two or more different species. Conventional antibodies for medical use produced using a mouse hybridoma problematically cause rejection in the immunity system after administered to human bodies because they are of mouse origin. In order to solve this problem, for example, a region such as the Fc region of the murine (rodent) antibody is humanized by a recombinant technique. This process can significantly reduce the risk of rejection.

Examples of the interspecific chimeric antibodies include anti-human CD2 antibodies, anti-CD20 receptor antibodies, and anti-TNF antibodies. Some of these are already on the market as drugs. In recent years, fully human antibodies in which the whole amino acid sequence is derived from a human antibody have been put into practical use as pharmaceutical products. Such antibodies are suitable as the antibody to be produced in the present invention.

The cytotoxic composition of the present invention can potentially be used in the field of pharmaceutical products. In the case that the cytotoxic composition of the present invention is used for an anti-tumor agent or an agent for ameliorating or treating a cancer, the composition preferably contains, as an active ingredient, an anti-cancer antibody that recognizes an antigen that is specifically expressed in cancer cells, and destroys the cancer cells. It is difficult to selectively remove cancer cells from healthy cells because they have few structural differences. However, as the presence of antigen proteins that are specifically expressed on the surface of cancer cells became apparent, diagnosis and medical treatment utilizing the ability of an antibody to recognize cancer cells have been being developed.

As the active ingredient of the cytotoxic composition of the present invention, an antibody for treating an autoimmune disease which neutralizes and inhibits an effector cell that reacts with an autoantigen is also preferable. In this case, the cytotoxic composition of the present invention can be used for an agent for ameliorating or treating an autoimmune disease such as an anti-rheumatic agent.

Such an antibody is required not only to recognize a target cell but also to inactivate the function of the target cell. In this function of the antibody, antibody-dependent cell mediated cytotoxicity (ADCC) activity and complement-dependent cytotoxicity (CDC) activity, which are described above, play important roles. Complement-dependent cytotoxicity activity is an activity in which an antibody binds to a target cell to activate the complement system and an enzyme of the complement effectively lyses the target cell. Recent studies on antibody drugs revealed that complement-dependent cytotoxicity activity plays an important role in the expression of anti-cancer effects.

Monoclonal antibodies with enhanced complement-dependent cytotoxicity activity compared with CHO cell-produced IgG antibodies and the like can more effectively destroy target cancer cells. Therefore, use of such monoclonal antibodies can lead to the reduction of administration dose, resulting in less side-effects. As a result, QOL of patients can be improved. The production costs can also be reduced, resulting in a smaller burden to patients. Antibody compositions suitably used in the cytotoxic composition of the present invention have CDC activity five or more times higher than that of the corresponding CHO cell-produced antibody compositions. Further, antibody compositions more suitably used for the cytotoxic composition of the present invention have CDC activity ten or more times higher than that of the corresponding CHO cell-produced antibody compositions.

Antibody compositions suitably used in the present invention have a fucose content of the complex N-glycoside-linked sugar chains of not more than one fifth of that of the corresponding CHO cell-derived antibodies, and more suitably the fucose content is not more than one tenth of that of the corresponding CHO cell-derived antibodies.

In order to regulate the expression of the transferred structural gene, a tissue-specific promoter sequence and the like are used. The term "tissue-specific promoter sequence" means a promoter sequence showing especially intense activity in specific avian tissues or cells. Use of the tissue-specific promoter sequence can advantageously reduce or eliminate the possibility of adverse influence of the expressed desired protein on the development and survival of avians. The tissue-specific promoter sequence is not particularly limited and examples thereof include oviduct-specific promoter sequences. Since the oviduct tissue becomes more active after sexual maturation, oviduct-specific promoter sequences tend to be strongly induced after sexual maturation in many cases.

Examples of the oviduct-specific promoter sequences include ovalbumin, ovotransferrin, ovomucoid, ovomucin, lysozyme, G2 globulin, G3 globulin, ovoinhibitor, ovoglycoprotein, ovoflavoprotein, ovomacroglobulin, cystatin, and avidin promoter sequences of avian origin. Use of these oviduct-specific promoter sequences is particularly preferable since the desired protein can be expressed in egg white at high levels.

Other examples of the usable promoter include non-tissue-specific promoters. The term "non-tissue-specific promoter sequence" refers to a promoter sequence that is not included in the tissue-specific promoter sequences. The non-tissue-specific promoter sequence is not particularly limited, and means that showing activity in substantially all avian somatic cells. The advantage of the non-tissue-specific promoters is that the expression or non-expression of the desired protein can be detected in the stage of nestlings since the desired protein is expressed in blood as well. The non-tissue-specific promoter sequence is not particularly limited and examples thereof include β-actin promoter sequence, EF1α promoter sequence, thymidine kinase promoter sequence, and viral promoter sequences such as simian virus 40 (SV40) promoter sequence, cytomegalovirus (CMV) promoter sequence, and Rous sarcoma virus (RSV) promoter sequence. Other examples thereof include non-tissue-specific inducible promoter sequences such as tetracycline-inducible promoter sequence.

The foreign gene may contain a transcriptional enhancer and/or a regulatory element. The term "transcription enhancer" means a sequence in a region of DNA or RNA, which promotes transcription from a promoter sequence but is unable to effect transcription by itself. Transcription enhancers, even when combined with a promoter sequence different from the original one with which they function, can function in many instances, and hence the combination thereof with a promoter sequence is not limited. The transcription enhancer is not particularly limited and examples thereof include the SV40 enhancer, CMV enhancer, thymidine kinase enhancer, steroid responsive element and lysozyme enhancer. The term "regulatory element" means a region on DNA or RNA, which contributes to transcriptional regulation and stabilization of transcribed RNA, and by itself cannot effect transcription. The regulatory element is not particularly limited and examples thereof include WPRE (woodchuck hepatitis virus-derived regulatory element, U.S. Pat. No. 6,136,597).

The foreign gene may also contain untranslated regions other than the above-described sequences.

A pseudotyped viral vector is prepared by utilizing a packaging cell, a helper virus or the like, and introduced into an early embryo, blood vessel or heart by a common microinjection technique (Bosselman, R. A. et al., Science, 243, 533, 1989). Other gene transfer techniques such as lipofection and electroporation techniques may be possible, but microinjection techniques are preferable in view of the operability and transfer efficiency.

The foreign gene is not particularly limited, and examples thereof include not only non-avian genes but also avian ones. Even a sequence included in the original genes of an individual subjected to transgenic production is referred to as a foreign gene since the gene is additionally transferred into the original genes of this individual.

The embryo is desirably infected with the replication defective retroviral vector not earlier than 24 hours, more desirably not earlier than 32 hours but not later than 72 hours, and further more desirably not earlier than 48 hours but not later than 64 hours after the start of incubation. Preferably, the site of infection, that is, the site into which the virus-containing fluid is introduced is the inside of the heart or blood vessel. For the purpose of production, of G0 transgenic chimeric avians with high gene transfer efficiency, it is preferable to transfer the gene at an early stage at which the cardiac pulsation can be observed (within 6 hours after the start of cardiac pulsation). This is because at this stage, the gene will be distributed to the whole body by means of blood circulation and the number of cells is small.

Microinjection techniques are techniques to introduce a virus-containing fluid directly into a target site with a tool such as a glass microtube with a fine tip under a microscope. In this study, the virus-containing fluid is introduced into a target site such as the heart or blood vessel, and therefore, the microinjection techniques are more preferable than other gene transfer techniques such as lipofection and electroporation techniques.

The avian used in the present invention is not particularly limited and is preferably a poultry utilizable as a farm animal. Examples of poultries include chickens, turkeys, ducks, ostriches, quails, and domestic ducks. Among them, chickens are particularly preferable since they are easily available and are fecund egg-laying species, their eggs are large, and the mass rearing method has been established.

G1 transgenic avians are obtained by mating G0 transgenic chimeric avians having a foreign gene in germ cells with wild-type avians, G0 transgenic chimeric avians or descendants thereof; and screening nestlings after hatch. In G0 transgenic chimeric avians, the probability of incorporation of the foreign gene into all cells is low, and in most cases, they are in a chimeric state in which there coexist cells different in genotype, that is, cells containing the transferred foreign gene and wild-type cells. In contrast, G1 transgenic avians contain the transferred gene uniformly in all somatic cells in preferable cases. Whether or not the gene is incorporated into somatic cells or germ cells can be confirmed by analyzing DNAs and RNAs from blood, somatic cells, sperms and eggs by techniques such as PCR, or can be confirmed based on the expression of the desired protein. The expression of the desired protein can be confirmed by techniques such as ELISA and electrophoresis, or by measuring the activity of the desired protein.

G2 and the subsequent generations of transgenic avians are produced by mating G1 transgenic avians. For example, mating between a G1 transgenic male and a wild-type female, a G1 transgenic female and a wild-type male, or G1 transgenic male and female may be possible. Further, backcrossing of a descendant with one of its parents is also possible. Among them, mating between a G1 male and a wild-type female is preferred in view of the efficiency since a single G1 male can be mated with a plurality of wild-type females.

The method for producing the desired protein of the present invention includes recovering the desired protein from the above-described transgenic avians, and more specifically includes extracting the substance from the blood and/or eggs of the produced transgenic avians and purifying it.

The purification is performed by a column technique or filtration using a solution prepared by diluting the transgenic avian eggs, in particular the egg white component, with pure water or an equilibrated salt solution. The dilution is carried out for the purpose of reducing the viscosity of egg white and smoothly carrying out a column technique. While a high dilution ratio is desired to reduce the viscosity, the increase in volume makes the recovery difficult. Therefore, the dilution ratio is preferably 2-10, and more preferably 5-6.

Examples of purification techniques to recover the desired antibody composition from the egg white solution include, but are not limited to, techniques using a protein A column, salting out, adsorption column chromatography, ion exchange column chromatography, gel filtration column chromatography and antibody column techniques. Any of these may be employed alone, or two or more of these may be employed in combination. Examples of the adsorption column chromatography include Blue Sepharose chromatography and heparin chromatography, and examples of the ion exchange column chromatography include anion exchange chromatography.

### EXAMPLES

In the following examples, an anti-TNF antibody was prepared by way of example. The examples show that an IgG antibody from eggs of transgenic avians has remarkably higher CDC activity than that of the corresponding CHO cell-derived IgG antibody. It should be noted that the present invention is not limited to these examples.

Gene manipulation was performed in accordance with the typical techniques (J. Samnrook, E. F. Fritsch, T. Maniatis; Molecular Cloning, A Laboratory Manual, 2nd Ed., Cold Spring Harbor Laboratory), unless otherwise specified. Cells were cultured in accordance with typical techniques, unless otherwise specified. Materials described with trade names were treated in accordance with the manuals attached thereto, unless otherwise specified.

### (Example 1)

### Construction of Fully Human Anti-TNF Antibody Expression Plasmid pMSCV/GΔALIH (HUMIRA (R))

Anti-human CD2 antibody pMSCV/GΔALIH (CD2) is disclosed in JOURNAL OF BIOSCIENCE AND BIOENGINEERING, Vol. 98, No. 4, 298-303, 2004.

The entire base sequence of the fully human anti-TNF antibody (Adalimumab, HUMIRA (R)) was synthesized based on the sequence information open to the public. The CD2 sequence of pMSCV/GΔALIH (CD2) was substituted with this synthesized sequence to provide pMSCV/GΔALIH (HUMIRA (R)).

### (Example 2)

### Preparation of Retroviral Vector Using pMSCV/GΔALIH (HUMIRA (R)) and pVSV-G

In the following description, the culture conditions are as follows, unless otherwise specified.
Medium: Dulbecco's modified Eagle's medium (DMEM) containing 10% fetal bovine serum (FBS), 50 units/ml penicillin, and streptomycin (product of Gibco)
Temperature: 37°C
CO₂ concentration: 5%
Endo Free Plasmid Maxi Kit (product of QIAGEN) was used to purify the plasmid DNA used in the retroviral vector.

In order to prepare a retroviral vector from the plasmid pMSCV/GΔALIH (HUMIPA (R)) constructed in Example 1, a collagen-coated culture dish (diameter: 100mm) was inoculated with GP293 packaging cells containing the gag and pol genes (5×10⁶ cells/dish (70% confluent), 90% confluent on the next day). On the next day, the culture medium was removed, and fresh culture medium (7.2 ml) and 25 mM chloroquine (10 µl, product of Sigma Corp.) were added, and the cells were cultured for further one hour. Lipofectamine 2000 solution (56 µl, product of Invitrogen Corp.) was suspended in Opti-MEMI medium (1.4 ml, product of Gibco), and the suspension was allowed to stand for five minutes at room temperature. pMSCV/GΔALIH (HUMIRA (R), 12 µg) and pVSV-G (12 µg) were suspended in Opti-MEMI medium (1.4 ml). The Lipofectamine 2000 solution and the plasmid DNA solution were mixed, and the mixture was allowed to stand at room temperature for 20 minutes. Then, the mixture was all added to the culture dish and cultured for six hours. After the six-hour culture, the medium was removed, fresh medium (9 ml) and 1 M HEPES Buffer Solution (200 µl, product of Gibco) were added, and the mixture was cultured for further 24 hours.

The culture supernatant was collected in a centrifuge tube through a 0.45-µm cellulose acetate filter (product of Advantec). The filtrate was centrifuged at 28,000 rpm (50,000 g) for 1.5 hours using an ultracentrifuge (CS100GXL, product of Hitachi Koki, Ltd.). The supernatant was removed, and 20 µl of TNE buffer (50 mM Tris-HCl (pH 7.8), 130 mM NaCl, and 1 mM EDTA) was added to the sediment, and the mixture was allowed to stand at 4°C overnight. The sediment was thoroughly suspended, and the suspension was centrifuged at 12,000 rpm for one minute using a small-scale high-speed centrifuge. The supernatant was passed through a 0.45-µm Durapore Ultrafree filter (product of Advantec) and the obtained filtrate was used as a virus-containing fluid.

### (Example 3)

### Virus Titer Measurement

The titer of the virus-containing fluid is defined as the number of infected cells after addition of the virus-containing fluid to NIH3T3 cells (ATCC CRL-1658). A 1-ml portion of the virus solution diluted at a dilution ratio of 10² to 10⁶ was added to 5×10⁴ NIH3T3 cells in each well (base area: about 9.4 cm²) of 6-well culture plates, and the proportion of cells expressing GFP (green fluorescent protein) gene (marker) was determined as the titer of the virus-containing fluid. If 4 colonies appear at a dilution ratio of 10⁶, the virus titer will be 4×10⁶ cfu/ml.

More specifically, 5×10⁴ NIH3T3 cells were inoculated into each well of 6-well culture plates on the day before the start of titer measurement and were cultured. On the next day, the culture medium of the cells in each well was replaced with 900 µl of medium containing 9 µg/ml of polybrene. The virus-containing fluid was diluted to 10⁻¹ to 10⁻⁵ with medium and a 100-µl portion of each dilution was added to each well for infection (final polybrene concentration: 8 µl/ml). After 4- to 6-hour culture, 1 ml of medium was further added to each well. On the next day, the medium was replaced with fresh one. Thereafter, the medium was replaced at 3- to 4-day intervals. About one week after the infection, the colonies containing expressed GFP were counted, and thus the titer was determined.

### (Example 4)

### Selection of Fully Human Anti-TNF Antibody Expression Stable Packaging Cell

On the day before viral infection, 1.5×10⁴ GP293 cells were inoculated into each well of 24-well culture plates and were cultured. On the day of viral infection, the culture medium in each well was replaced with 1 ml of medium containing 10 µg/ml of polybrene, and the cells were infected with the virus-containing fluid prepared in Example 2. Thereafter, the cells were cloned by limiting dilution. More specifically, on the next day, the cells were diluted to 10 cells/ml . A 100-µl portion of the cell dilution was inoculated into each well of 96-well culture plates (so that one cell might be contained in each well). A cell line showing a high cell growth rate and morphologically close to GP293 cells was selected. Thus, a fully human anti-TNF antibody expression stable packaging cell clone was obtained.

### (Example 5)

### Preparation of Retroviral Vector Using Fully Human Anti-TNF Antibody Expression Stable Packaging Cell and pVSV-G

A collagen-coated culture dish (diameter: 100 mm) was inoculated with the fully human anti-TNF antibody expression stable packaging cells (5×10⁶ cells (70% confluent), 90% confluent on the next day). On the next day, the culture medium was removed, and fresh medium (7.2 ml) and 25 mM chloroquine (10 µl) were added, and the cells were cultured for further one hour. Lipofectamine 2000 solution (56 µl) was suspended in Opti-MEMI medium (1.4 ml), and the suspension was allowed to stand at room temperature for five minutes. pVSV-G (12 µg) was suspended in Opti-MEMI medium (1.4 ml). The Lipofectamine 2000 solution and the plasmid DNA solution were mixed, and the mixture was allowed to stand at room temperature for 20 minutes. The mixture was all added to the culture dish and cultured for six hours. After the six-hour culture, the medium was removed, fresh medium (9 ml) and 1 M HEPES Buffer Solution (200 µl) were added, and the mixture was cultured for further 24 hours.

The culture supernatant was passed through a 0.45-µm cellulose acetate filter, and the filtrate was collected in a centrifuge tube. The filtrate was centrifuged at 28,000 rpm (50,000 g) for 1.5 hours using an ultracentrifuge. The supernatant was removed, TNE buffer (20 µl) was added to the sediment, and the mixture was allowed to stand at 4°C overnight. The sediment was thoroughly suspended, and the suspension was centrifuged at 12,000 rpm for one minute using a small-scale high-speed centrifuge. The supernatant was passed through a 0.45-µm Durapore Ultrafree filter (product of Advantec), and the obtained filtrate was used as a virus-containing fluid. Thus, the virus-containing fluid having a titer of 10⁸ cfu/ml or higher was obtained.

### (Example 6)

### Production of Fully Human Anti-TNF Antibody Producing Transgenic Chicken by Microinjection of Retroviral Vector into Chicken Embryo and Artificial Hatching

Microinjection and artificial hatching were carried out under sterile conditions. The surface of fertilized chicken eggs (Shiroyama Shukeijo (Shiroyama Chicken Farm)) was disinfected with a disinfectant (product of Showa Furanki) and ethanol. An incubator (P-008 (B), product of Showa Furanki) was adjusted to a temperature of 38°C and a humidity of 50% to 60%, and the eggs were incubated from the start of power supply (incubation start time, 0 hour). Thereafter, the eggs were turned at 90° at 15-minute intervals.

After the lapse of about 55 hours from the start of incubation, the incubator was readjusted so that the eggs were turned at 30° at 30-minute intervals. The eggs were taken out of the incubator, and the sharper end of each egg was cut off to provide a circular opening with a diameter of 3.5 cm with a minirouter (product of Proxxon) equipped with a diamond edge (edge diameter: 2.0 mm; shaft diameter: 2.35 mm). The sharper end of each Double-yolked egg (Shiroyama Shukeijo (Shiroyama Chicken Farm)) was cut off to provide a circular opening with a diameter of 4.5 cm, and the contents were discarded. Into the remaining eggshells, the contents of the fertilized eggs were transferred, and the embryos were shifted upward with the inner cylinder of a syringe. The virus-containing fluid was poured into Femtotips II (product of Eppendorf) under a stereoscopic microscope system (SZX12, product of Olympus Corp.), and about 2 µl of the virus solution was microinjected using FemtoJet (product of Eppendorf). Each opening was sealed with Saran Wrap (product of Asahi Kasei Corp.) cut to a size of about 8×8 cm², using egg white as an adhesive, and the eggs were returned to the incubator for further incubation. On the 20th day after the start of incubation, about 20 holes were bored in the Saran Wrap using a 20G syringe needle, and oxygen was fed to the incubator at 60 cc/min for hatching. When the chicks began pecking, the eggshells were broken to allow the chicks to hatch. The hatchability in this artificial hatching was 7% to 30%.

### (Example 7)

### Recovery of IgG Antibody from Transgenic Chicken Egg

The hatched chicks were fed for growth. The feeds used were SX Safety for young chicks and Neo-Safety 17 (products of Toyohashi Feed Mills Co., Ltd.). The IgG antibody was extracted from egg white of eggs of the transgenic chickens. The egg white was pretreated to be wholly homogeneous by an ultrasonic or physical technique. The IgG antibody was prepared by a common column purification technique using a protein A column. The prepared IgG antibody was stored frozen at -80°C until assaying. It is preferable to thaw the antibody rapidly at 37°C and avoid repeated freeze-thaw cycles.

### (Example 8)

### Quantification of Immunoglobulin Molecule Concentration by ELISA

The amount of IgG antibody expression in the egg white prepared in Example 7 was measured by Enzyme-Linked Immunosorbent Assay (ELISA). The amount of produced antibody was calculated based on a standard used for the assay.

The measurement was performed in accordance with the typical method (Immunochemistry, 8, 871-874 (1971)).

A rabbit anti-human IgG (Fc-specific) antibody (Organon Teknika, Durham, NC, USA) and a peroxidase-labelled rabbit anti-human IgG antibody (Organon Teknika, Durham, NC, USA) were used as a primary antibody and secondary antibody, respectively. Color was developed using O-phenylenediamine as a detection substrate, and the absorbance was determined with a plate reader. The standard used for quantification was a human IgGl (Athens Research and Technology, Athens, GA, USA).

### (Example 9)

The CDC activity level of the transgenic-chicken egg white-derived fully human anti-TNF antibody prepared through Example 1 to 7 was compared with the CDC activity level of a CHO cell-produced fully human antibody.

To each well of a 24-well plate was added 1 ml (5×10⁵ cells/well) of T cells (Jurkat cells (ADCC No. TIB-152) or Raji cells (ATCC No. CCL-86)) containing an expressed TNF antigen. Further, 10% human serum RPMI1640 (product of Nissui) and the antibody sample (0.01, 0.1, 1, or 10 µg/ml) were added thereto, and the cells were cultured for three hours at 37°C. Then, the cells were stained with PI (propidium iodide) and analyzed by FACS (Fluorescence Activated Cell Sorting) to determine the number of viable cells. The mortality of the target cell was calculated as follows.

### Mortality of target cell = (total number of cells - number of viable cells) / total number of cells (%)

As shown in Fig. 1, the fully human anti-TNF antibody derived from egg white of the transgenic chickens produced an equivalent killing effect on the target cell at a concentration of 1/10 of that of the CHO cell-derived fully human anti-TNF antibody. Namely, the results show that CDC activity of the fully human anti-TNF antibody derived from egg white of the transgenic chickens is 10 times higher than that of the CHO cell-derived fully human anti-TNF antibody. The CHO cell-derived fully human anti-TNF antibody used herein was a commercially available antibody, adalimumab (trade name: HUMIRA (R)).

## Claims

1. A cytotoxic composition comprising an IgG antibody as an active ingredient, the IgG antibody being obtainable from an egg of a transgenic avian that contains a gene encoding the IgG antibody.

2. The cytotoxic composition according to claim 1, wherein the active ingredient is an antibody obtainable from egg white.

3. The cytotoxic composition according to claim 1 or 2, wherein the IgG antibody has a constant region of human IgG class.

4. The cytotoxic composition according to any one of claims 1 to 3,
wherein the transgenic avian is a transgenic poultry.

5. The cytotoxic composition according to any one of claims 1 to 4,
wherein the fucose content of complex N-glycoside-linked sugar chains of the antibody is not more than one fifth of that of the corresponding CHO cell-derived antibody.

6. The cytotoxic composition according to any one of claims 1 to 5,
wherein CDC activity of the antibody is five or more times higher than that of the corresponding CHO cell-derived antibody.

7. The cytotoxic composition according to any one of claims 1 to 6,
wherein the IgG antibody recognizes an antigen specifically expressed in a cancer cell.

8. An agent for ameliorating or treating a cancer, comprising the cytotoxic composition according to any one of claims 1 to 7.

9. The cytotoxic composition according to any one of claims 1 to 6,
wherein the IgG antibody neutralizes and inhibits an effector cell that reacts with an autoantigen.

10. An agent for ameliorating or treating an autoimmune disease, comprising the cytotoxic composition according to any one of claims 1 to 6 and 9.
